# EUROPEAN PATENT APPLICATION

(11) **EP 3 901 167 A1**
(43) Date of publication of application: **27.10.2021**
(21) Application number: 19900235.3
(22) Date of filing: 30.09.2019
(51) Int. Cl.: C07K 14/55

(54) **NOVEL IMMUNOSUPPRESSIVE INTERLEUKIN 2**

(30) Priority: 21.12.2018 KR 20180167797
(71) Applicant: Hanmi Pharm. Co., Ltd., Hwaseong-si, Gyeonggi-do 18536 (KR)
(72) Inventor: OH, Euh Lim, Hwaseong-si, Gyeonggi-do 18469 (KR); LEE, Hyo Ran, Hwaseong-si, Gyeonggi-do 18469 (KR); KIM, Jin Young, Hwaseong-si, Gyeonggi-do 18469 (KR); HEO, Yong Ho, Hwaseong-si, Gyeonggi-do 18469 (KR)
(74) Representative: Mewburn Ellis LLP
(86) International application number: PCT/KR2019/012716
(87) International publication number: WO 2020/130300

(57) **Abstract**

The present invention relates to an interleukin-2 analogue, which has altered binding affinity for interleukin-2 alpha receptor and interleukin-2 beta receptor, compared to the wild type.

## Description

### [Technical Field]

The present invention relates to novel interleukin-2 analogs.

### [Background Art]

Interleukin-2 is an important immunostimulator with a molecular weight of about 15 kDa composed of a total of 133 amino acid residues, and activates various cells of the immune system, including T cells and B cells. The high efficacy of interleukin-2 as an immunostimulator can be used to treat various immune-related disease symptoms, including cancer and AIDS (Korean Patent Publication No. 10-2017-0070091). Interleukin-2 (brand name: Proleukin) is currently an FDA-approved drug for the treatment of metastatic renal cell carcinoma and metastatic melanoma. However, the severe toxicity associated with high-dose interleukin-2 therapy limits the number of applicable patients, and only a few suitable patients are actually receiving such therapy. The toxicity associated with interleukin-2 includes severe fever, nausea, vomiting, vascular leak, severe hypotension, pulmonary edema, and vascular leak syndrome causing liver damage.

The interleukin-2 has three subunit receptors. The subunits are the alpha chain (IL-2Rα or CD25), beta chain (IL-2Rβ or CD122), and gamma chain (IL-2Rγ or CD132), and interleukin-2 can exert its pleiotropic functions by binding to each combination of the receptor subunits. The single interleukin-2 alpha receptor is termed the low-affinity interleukin-2 receptor and does not participate in signaling. A complex of interleukin-2 beta receptor and gamma receptor binds interleukin-2 with intermediate affinity. A complex of interleukin-2 alpha receptor, beta receptor, and gamma receptor binds interleukin-2 with high affinity. The complex of interleukin-2 beta receptor and gamma receptor is needed in effective signal transduction through the activation of kinase on multiple signaling pathways. In particular, the combination of interleukin-2 beta receptor and gamma receptor is prominent in CD8+ cells and natural killer (NK) cells. In addition, the complex of interleukin-2 alpha receptor, beta receptor, and gamma receptor, although usually found in CD4+ regulatory T cells (T_{reg}), has also recently been found in activated T cells.

Despite the potential of interleukin-2 as a medicine for various immune-related disease symptoms, drugs capable of lowering the dose while reducing toxicity and side effects are insufficient, and thus further research is needed on novel and improved drugs.

### [Disclosure]

### [Technical Problem]

An object of the present invention is to provide a novel interleukin-2 analog having changes in binding affinity to interleukin alpha receptor and beta receptor compared with natural interleukin-2.

The interleukin-2 analog, compared with natural interleukin-2, may have an increase in binding affinity to interleukin-2 alpha receptor and a decrease or increase in binding affinity to interleukin beta receptor.

Another object of the present invention is to provide a method for preparing the interleukin-2 analog.

Still another object of the present invention is to provide a method for changing binding affinity to interleukin-2 alpha receptor and interleukin-2 beta receptor compared with natural interleukin-2, the method including altering at least one amino acid in the natural interleukin-2.

### [Technical Solution]

An aspect of the present invention provides an interleukin-2 analog. The interleukin-2 (IL-2) analog is an interleukin analog having changes in binding affinity to interleukin-2 alpha receptor and/or binding affinity to interleukin-2 beta receptor compared with the natural form.

In an embodiment, the changes in binding affinity are an increase in binding affinity to interleukin-2 alpha receptor and a decrease or increase in binding affinity to interleukin-2 beta receptor.

In an embodiment of any one of the preceding embodiments, the decrease or increase in binding affinity to interleukin-2 beta receptor shows 10% to 200% compared to the binding affinity of the natural interleukin-2.

In an embodiment of any one of the preceding embodiments, the interleukin-2 analog has an alteration selected from the group consisting of substitution, addition, deletion, modification, and a combination thereof of at least one amino acid in the natural interleukin-2.

In an embodiment of any one of the preceding embodiments, the interleukin-2 analog has an alteration of at least one amino acid selected from the group consisting of the amino acids at positions 1, 61, 62, 68, and 125 in the natural interleukin-2.

The interleukin-2 analog according to any one of the preceding embodiments has a substitution of at least one amino acid with another amino acid or a deletion of at least one amino acid, the at least one amino acid being selected from the group consisting of the amino acids at positions 1, 61, 62, 68, and 125 in the natural interleukin-2.

The interleukin-2 analog according to any one of the preceding embodiments is selected from: (a) an interleukin-2 analog having an alteration of the amino acid at position 1 in the natural interleukin-2; (b) an interleukin-2 analog having an alteration of the amino acid at position 61 to another amino acid in the natural interleukin-2; (c) an interleukin-2 analog having an alteration of the amino acid at position 62 to another amino acid in the natural interleukin-2; (d) an interleukin-2 analog having an alteration of the amino acid at position 68 to another amino acid in the natural interleukin-2; and (e) an interleukin-2 analog having an alteration of the amino acid at position 125 to another amino acid in the natural interleukin-2.

The interleukin-2 analog according to any one of the preceding embodiments is selected from: (a) an interleukin-2 analog having a deletion of the amino acid at position 1 and a substitution of the amino acid at position 125 with another amino acid in the natural interleukin-2; (b) an interleukin-2 analog having a deletion of the amino acid at position 1 and substitutions of the amino acids at positions 125 and 61 with other amino acids in the natural interleukin-2; (c) an interleukin-2 analog having a deletion of the amino acid at position 1 and substitutions of the amino acids at positions 125 and 62 with other amino acids in the natural interleukin-2; and (d) an interleukin-2 analog having a deletion of the amino acid at position 1 and substitutions of the amino acids at positions 125 and 68 with other amino acids in the natural interleukin-2.

The interleukin-2 analog according to any one of the preceding embodiments is selected from: (a) an interleukin-2 analog having a deletion of the amino acid at position 1, a substitution of the amino acid at position 125 with serine, and a substitution of the amino acid at position 61 with aspartic acid in the natural interleukin-2; (b) an interleukin-2 analog having a deletion of the amino acid at position 1, a substitution of the amino acid at position 125 with serine, and a substitution of the amino acid at position 61 with glutamine in the natural interleukin-2; (c) an interleukin-2 analog having a deletion of the amino acid at position 1, a substitution of the amino acid at position 125 with serine, and a substitution of the amino acid at position 62 with glutamine in the natural interleukin-2; and (d) an interleukin-2 analog having a deletion of the amino acid at position 1, a substitution of the amino acid at position 125 with serine, and a substitution of the amino acid at position 68 with aspartic acid in the natural interleukin-2.

The interleukin-2 analog according to any one of the preceding embodiments is selected from the group consisting of SEQ ID NOS: 16, 18, 20, and 22.

Another aspect for implementing the present invention provides: an isolated nucleic acid encoding the interleukin-2 analog; a recombinant expression vector including the nucleic acid; and a transformant including the vector.

Still another aspect for implementing the present invention provides a method for preparing the interleukin-2 analog.

Another aspect for implementing the present invention provides a method for changing binding affinity to interleukin-2 alpha receptor and interleukin-2 beta receptor compared with natural interleukin-2, the method including altering at least one amino acid in the natural interleukin-2.

In an embodiment, the changing of the binding affinity is increasing the binding affinity to interleukin-2 alpha receptor and decreasing or increasing the binding affinity to interleukin-2 beta receptor.

In the method according to any one of the preceding embodiments, the altering is altering at least one amino acid to another amino acid, the at least one amino acid being selected from the group consisting of amino acids at positions 1, 61, 62, 68, and 125 in the natural interleukin-2.

In the method according to any one of the preceding embodiments, the altering is: (a) deleting the amino acid at position 1 and substituting the amino acids at positions 125 and 61 with other amino acids in the natural interleukin-2; (c) deleting the amino acid at position 1 and substituting the amino acids at positions 125 and 62 with other amino acids in the natural interleukin-2; or (d) deleting the amino acid at position 1 and substituting the amino acids at positions 125 and 68 with other amino acids in the natural interleukin-2.

### [Advantageous Effects]

The interleukin-2 analogs according to the present invention are analogs having changes in binding affinity to interleukin-2 alpha receptor and/or interleukin-2 beta receptor *in vivo* compared with natural interleukin-2, and can be used for various purposes.

### [Brief Description of Drawings]

FIG. 1 shows the results of purity analysis after purification of interleukin-2 analogs (RPC: RP-HPLC).
FIG. 2 shows the results of verifying binding affinity of interleukin-2 analogs to interleukin-2 alpha receptor: (A) interleukin-2 analog 1; (B) interleukin-2 analog 2; (C) interleukin analog 3; (D) interleukin analog 4; and (E) interleukin analog 5.
FIG. 3 shows the results of verifying binding affinity of interleukin-2 analogs to interleukin-2 beta receptor: (A) interleukin-2 analog 1; (B) interleukin-2 analog 2; (C) interleukin analog 3; (D) interleukin analog 4; and (E) interleukin analog 5.

### [Detailed Description of the Invention]

Hereinafter, the detailed description of the present invention is provided as below. Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description below.

Throughout the specification, conventional 1-letter and 3-letter codes for amino acids are used. The amino acids mentioned in abbreviations herein are described according to the IUPAC-IUB rules.
alanine A, arginine R
asparagine N, aspartic acid D
cysteine C, glutamic acid E
glutamine Q, glycine G
histidine H, isoleucine I
leucine L, lysine K
methionine M, phenylalanine F
proline P, serine S
threonine T, tryptophan W
tyrosine Y, valine V

An aspect of the present invention provides an interleukin-2 analog. The interleukin-2 analog of the present invention is characterized by having changes in binding affinity to interleukin-2 alpha receptor and/or binding affinity to interleukin-2 beta receptor compared with natural interleukin-2. Specifically, the changes in binding affinity are characterized by an increase in binding affinity to interleukin-2 alpha receptor and a decrease or increase in binding affinity to interleukin-2 beta receptor.

As used herein, the term "interleukin-2 (IL-2)" refers to a type of cytokine for signaling in the *in vivo* immune system and to an immunomodulator. Interleukin-2 is generally an important immunostimulator of about 15 kDa, the sequence information of which is available from known databases, such as the NCBI GenBank database.

As used herein, the term "interleukin-2 analog" refers to an analog having an alteration of at least one amino acid in the sequence of the natural form, especially one having a change in binding affinity to interleukin-2 receptor compared with the natural form, and may be an interleukin-2 analog having an alteration in an amino acid of interleukin-2, whereby the binding affinity to the alpha receptor is increased and/or the binding affinity to the beta receptor is decreased or increased.

The interleukin-2 analog may be a non-naturally occurring analog.

The alteration of at least one amino acid in the sequence of the natural form may be an alteration, selected from the group consisting of substitution, addition, deletion, modification, and a combination thereof, of at least one amino acid in the natural interleukin-2.

The interleukin-2 analog of the present invention may be an analog having a substitution of at least one amino acid selected from the group consisting of amino acids at positions 1, 61, 62, 68, and 125 in the natural interleukin-2 with another amino acid, or a deletion thereof, but is not limited thereto.

Interleukin-2 peptides having substitution, addition, deletion, modification, and the like of amino acid residues, which are known in the art and could be performed so as to increase the stability and half-life of the peptides without influencing activity of the peptides, as well as amino acid residues at the above alteration positons, are also included in the scope of the present invention.

The natural interleukin-2 may be human interleukin-2, and may be SEQ ID NO: 23, but is not limited thereto. The natural interleukin-2 may be Proleukin®, which is commercially available, besides human interleukin-2, and may be an object of comparison with the interleukin-2 analog of the present invention regarding binding affinity to interleukin-2 alpha receptor and interleukin-2 beta receptor, but is not limited thereto. An example thereof may be SEQ ID NO: 14, but is not limited thereto.

In the present invention, the interleukin-2 analog may be selected from: (a) an interleukin-2 analog having an alteration of the amino acid at position 1 in the natural interleukin-2; (b) an interleukin-2 analog having an alteration of the amino acid at position 61 to another amino acid in the natural interleukin-2; (c) an interleukin-2 analog having an alteration of the amino acid at position 62 to another amino acid in the natural interleukin-2; (d) an interleukin-2 analog having an alteration of the amino acid at position 68 to another amino acid in the natural interleukin-2; and (e) an interleukin-2 analog having an alteration of the amino acid at position 125 to another amino acid in the natural interleukin-2.

As another example, the interleukin-2 analog may be any one selected from: (a) an interleukin-2 analog having a deletion of the amino acid at position 1 and a substitution of the amino acid at position 125 with another amino acid in the natural interleukin-2; (b) an interleukin-2 analog having a deletion of the amino acid at position 1 and substitutions of the amino acids at positions 125 and 61 with other amino acids in the natural interleukin-2; (c) an interleukin-2 analog having a deletion of the amino acid at position 1 and substitutions of the amino acids at positions 125 and 62 with other amino acids in the natural interleukin-2; and (d) an interleukin-2 analog having a deletion of the amino acid at position 1 and substitutions of the amino acids at positions 125 and 68 with other amino acids in the natural interleukin-2. However, the interleukin-2 analog includes, without limitation, any interleukin-2 analog having changes in binding affinity to interleukin-2 alpha receptor and/or binding affinity to interleukin-2 beta receptor compared with the natural interleukin-2. The changes in binding affinity are as described above.

As still another example, the interleukin-2 analog may be any one selected from: (a) an interleukin-2 analog having a deletion of the amino acid at position 1, a substitution of the amino acid at position 125 with serine, and a substitution of the amino acid at position 61 with aspartic acid in the natural interleukin-2; (b) an interleukin-2 analog having a deletion of the amino acid at position 1, a substitution of the amino acid at position 125 with serine, and a substitution of the amino acid at position 61 with glutamine in the natural interleukin-2; (c) an interleukin-2 analog having a deletion of the amino acid at position 1, a substitution of the amino acid at position 125 with serine, and a substitution of the amino acid at position 62 with glutamine in the natural interleukin-2; and (d) an interleukin-2 analog having a deletion of the amino acid at position 1, a substitution of the amino acid at position 125 with serine, and a substitution of the amino acid at position 68 with aspartic acid in the natural interleukin-2.

As still another example, the interleukin-2 analog may be selected from the group consisting of SEQ ID NOS: 16, 18, 20, and 22, but is not limited thereto.

Although described as "interleukin-2 analog consisting of a particular sequence number" herein, such expression does not exclude any addition of nonsense sequences, naturally occurring mutations, or silent mutations thereof upstream or downstream of the amino acid sequence of the corresponding sequence number as long as the analog has the same or equivalent activity as the interleukin-2 analog consisting of the amino acid sequence of the corresponding sequence number, and it is obvious that such a sequence addition or mutation is also within the scope of the present invention.

The above description can be applied to other embodiments or aspects of the present invention, but is not limited thereto.

The interleukin-2 analogs having alterations of amino acids at the above positions have an increase in binding affinity to interleukin-2 alpha receptor and a decrease or increase in binding affinity to interleukin-2 beta receptor, and thus can be used as novel interleukin-2 substitutes having a change in *in vitro* activity.

In addition, such an alteration for preparation of the interleukin-2 analogs encompasses all of: alterations using L-type or D-type amino acids and/or nonnatural amino acids; and/or alterations through modifications of the sequence of the natural form, for example, an alteration of a side chain functional group, intramolecular covalent bonding (e.g., a ring formation between side chains), methylation, acylation, ubiquitination, phosphorylation, aminohexanation, biotinylation, and the like.

In addition, such an alteration also encompasses all of additions of one or more amino acids to the amino and/or carboxy terminus of natural interleukin-2.

The amino acids used for substitution or addition may be not only the 20 amino acids commonly found in human proteins, but also atypical amino acids or those which do not occur naturally. Commercial sources of atypical amino acids may include Sigma-Aldrich, ChemPep, and Genzyme Pharmaceuticals. The peptides including these amino acids and typical peptide sequences can be synthesized and purchased through commercial suppliers, for example, American Peptide Company or Bachem in the USA, or Anygen in Korea.

Amino acid derivatives may also be accessible in a similar manner; for example, 4-imidazoacetic acid and the like may be used.

In addition, the interleukin-2 analog (interleukin-2 analog peptide) according to the present invention may be in a variant form in which the N-terminus and/or C-terminus of the peptide is chemically modified or protected by an organic group, or an amino acid is added to a terminus or the like of the peptide, for the protection from *in vivo* proteases and the increase in stability.

In particular, chemically synthesized peptides have electrically charged Nand C-termini, and thus for elimination of these charges, the N-terminus may be acetylated and/or the C-terminus amidated, but the peptide is not particularly limited thereto.

The interleukin-2 analog according to the present invention is in the form of a peptide, and thus encompasses all of the peptide itself, a salt thereof (e.g., a pharmaceutically acceptable salt of the peptide), or a solvate thereof. The peptide may also be in any pharmaceutically acceptable form.

The type of the salt is not particularly limited. The salt is preferably in the form that is safe and effective to a subject, e.g., a mammal, but is not particularly limited thereto.

The term "pharmaceutically acceptable" means a material which can be effectively used for a desired use without causing excessive toxicity, irritation, allergic responses, or the like, within the scope of medical and pharmaceutical decisions.

As used herein, the term "pharmaceutically acceptable salt" refers to a salt derived from a pharmaceutically acceptable inorganic acid, organic acid, or base. Suitable examples of the acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Suitable examples of the salt derived from a base may include alkali metals, such as sodium and potassium, alkaline earth metals, such as magnesium and the like, ammonium, and the like.

As used herein, the term "solvate" refers to a complex formed between the peptide or the salt thereof according to the present invention and a solvent molecule.

In the present invention, the binding affinity of any interleukin-2 analog to the natural interleukin-2 receptor may be measured by using various known techniques as methods of measuring affinity to receptors. For example, surface plasmon resonance (SPR) may be used, but the technique is not limited thereto.

More specifically, the interleukin-2 analog of the present invention may exhibit binding affinity to interleukin-2 alpha receptor which is about 101% or more, about 150% or more, about 200% or more, about 250% or more, about 300% or more, about 350% or more, about 400% or more, about 450% or more, about 500% or more, about 550% or more, about 600% or more, about 650% or more, about 700% or more, about 750% or more, about 800% or more, about 850% or more, about 900% or more, about 950% or more, or about 1000% or more compared with the binding affinity (100%) of the natural interleukin-2 to interleukin-2 alpha receptor. In a specific embodiment of the present invention, the binding affinity of the interleukin-2 analog to natural interleukin-2 alpha receptor is preferably about 150% to about 1000% of the binding affinity of the natural interleukin-2, but the values are not limited, and any binding affinity falls within the scope of the present invention as long as the binding affinity is stronger than the binding affinity of the natural interleukin-2.

More specifically, the binding affinity of the interleukin-2 analog of the present invention to interleukin-2 beta receptor may be decreased or increased compared with the binding affinity (100%) of the natural interleukin-2. The range of the decrease or increase in binding affinity to interleukin-2 beta receptor may be 10% to 200% compared with the binding affinity to the natural interleukin-2.

As an example, with respect to the decrease compared with the binding affinity of the natural interleukin-2 to interleukin-2 beta receptor, the interleukin-2 analog of the present invention may exhibit binding affinity to interleukin-2 beta receptor which is about 99% or less, about 95% or less, about 90% or less, about 85% or less, about 80% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, or about 10% or less of the binding affinity of the natural interleukin-2. As still another example, with respect to the increase compared with the binding affinity of the natural interleukin-2 to interleukin-2 beta receptor, the interleukin-2 analog of the present invention may exhibit binding affinity to interleukin-2 beta receptor which is about 101% or more, about 105% or more, about 110% or more, about 120% or more, about 130% or more, about 140% or more, about 150% or more, about 160% or more, about 170% or more, about 180% or more, about 190% or more, about 195% or more, or about 200% or more of the binding affinity of the natural interleukin-2, but the values are not limited, and any binding affinity falls within the scope of the present invention as long as the binding affinity is increased compared with the binding affinity of the natural interleukin-2. In a specific embodiment of the present invention, the binding affinity of the interleukin-2 analog to natural interleukin-2 beta receptor is preferably about 10% to about 200% of the binding affinity of the natural interleukin-2, but the values are not limited, and any binding affinity falls within the scope of the present invention as long as the binding affinity is decreased or increased compared with the binding affinity of the natural interleukin-2.

As used herein, the term "about" refers to a range including all of ±0.5, ±0.4, ±0.3, ±0.2, ±0.1, and the like, and includes all of the values in the range equivalent or similar to those stated after this term, but is not limited thereto.

The interleukin-2 analog of the present invention is characterized by having an increase in binding affinity to interleukin-2 alpha receptor and a decrease or increase in binding affinity to interleukin-2 beta receptor, compared with natural interleukin-2.

In a specific exemplary embodiment of the present invention, in order to prepare the interleukin-2 analogs of the present invention, interleukin-2 analogs having alterations introduced on the basis of the natural interleukin-2 were prepared.

Specifically, the interleukin-2 analog was prepared by using as a backbone the nucleic acid sequence encoding the interleukin-2 delA1,C125S analog having SEQ ID NO: 13, which is the nucleic acid sequence encoding analog 1 having a deletion of alanine as the amino acid at position 1 and a substitution of cysteine as the amino acid at position 125 with serine in the natural interleukin-2.

The other interleukin-2 analogs containing the sequence of analog 1 were prepared: the interleukin-2 delA1,C125S,E61D analog having SEQ ID NO: 15, which is the DNA sequence of analog 2 having a substitution of glutamic acid as the amino acid at position 61 with aspartic acid in the natural interleukin-2; the interleukin-2 delA1,C125S,E61Q analog having SEQ ID NO: 17, which is the DNA sequence of analog 3 having a substitution of glutamic acid as the amino acid at position 61 with glutamine in the natural interleukin-2; the interleukin-2 delA1,C125S,E62Q analog having SEQ ID NO: 19, which is the DNA sequence of analog 4 having a substitution of glutamic acid as the amino acid at position 62 with aspartic acid in the natural interleukin-2; and the interleukin-2 delA1,C125S,E68D analog having SEQ ID NO: 21, which is the DNA sequence of analog 5 having a substitution of glutamic acid as the amino acid at position 68 with aspartic acid in the natural interleukin-2.

Another aspect for implementing the present invention provides a nucleic acid encoding the interleukin-2 analog, a recombinant expression vector including the nucleic acid, and a transformant including the nucleic acid or recombinant expression vector.

The recombinant vector according to the present invention may be typically constructed as a vector for cloning or a vector for expression, and may be constructed as a vector for using a prokaryotic cell or eukaryotic cell as a host cell.

As used herein, the term "vector" refers to a recombinant vector capable of expressing a target protein in an appropriate host cell and to a nucleic acid construct including essential regulatory factors operatively linked to enable the expression of a nucleic acid insert. In the present invention, a recombinant vector including a nucleic acid encoding an interleukin-2 analog may be constructed, wherein the interleukin-2 analog of the present invention can be obtained by transformation or transfection of the recombinant vector into a host cell.

As used herein, the term "transformation" refers to a process of introducing DNA into a host cell to make the DNA replicable therein as a chromosomal factor or by completion of chromosomal integration, and means a phenomenon of artificially causing a genetic change by introducing exogenous DNA into a cell. The host suitable in the present invention is not particularly limited as long as the host enables the expression of the nucleic acid of the present invention. Specific examples of the host usable in the present invention include: bacteria of the genus *Escherichia* such as *E. coli;* bacteria of the genus *Bacillus* such as *Bacillus subtilis;* bacteria of the genus *Pseudomonas* such as *Pseudomonas putida;* yeasts such as *Pichia pastoris, Saccharomyces cerevisiae,* and *Schizosaccharomyces pombe;* insect cells such as *Spodoptera frugiperda* (SF9); and animal cells such as CHO, COS, and BSC.

Another aspect for implementing the present invention provides a method for preparing an interleukin-2 analog by using the transformant.

Specifically, the present invention provides a method for preparing the interleukin-2 analog, the method including:
a) culturing a transformant containing a nucleic acid encoding the interleukin-2 analog to express the interleukin-2 analog; and
b) isolating and purifying the expressed interleukin-2 analog.

A medium used to culture the transformant in the present invention needs to meet the requirements for culture of host cells in an appropriate manner. A carbon source that may be contained in the medium for growth of host cells may be appropriately selected through the decision of a skilled person in the art according to the type of transformant prepared, and appropriate culture conditions may be adopted to control the time and amount of culture.

Examples of a usable sugar source may include: sugars and carbohydrates, such as glucose, saccharose, lactose, fructose, maltose, starch, and cellulose; oils and fats, such as soybean oil, sunflower oil, castor oil, and coconut oil; fatty acids, such as palmitic acid, stearic acid, and linoleic acid; alcohols, such as glycerol and ethanol; and organic acids, such as acetic acid. These materials may be used alone or in combination.

Examples of a usable nitrogen source may include: peptones, yeast extracts, meat juices, malt extracts, corn steep liquor, soybean flour, urea, and inorganic compounds, such as ammonium sulfate, ammonium chloride, ammonium phosphate, ammonium carbonate, and ammonium nitrate. These nitrogen sources may also be used separately or in a mixture thereof.

Examples of a usable phosphorous source may include potassium dihydrogen phosphate, dipotassium hydrogen phosphate, or corresponding sodium-containing salts. In addition, culture media may contain a metal salt, such as magnesium sulfate or iron sulfate, which are necessary for growth.

In addition to the above materials, essential growth materials, such as amino acids and vitamins, may be used. Additionally, precursors appropriate for culture media may be used. The above raw materials may be added batchwise or continuously to the culture in an appropriate manner during culturing. The pH of the culture may be adjusted by using a basic compound in an appropriate manner, such as sodium hydroxide, potassium hydroxide, or ammonia, or an acidic compound, such as phosphoric acid or sulfuric acid. In addition, a defoaming agent, such as fatty acid polyglycol ester, may be added to prevent foam generation. In order to maintain an aerobic state, oxygen or an oxygencontaining gas (e.g., air) may be injected into the culture.

The transformant according to the present invention may be cultured at a temperature of 20°C to 45°C, and preferably 25°C to 40°C. The culture is continued until the maximum amount of production of a desired interleukin-2 analog is obtained, and for this purpose, the culture may usually be continued for 10 to 160 hours.

As described above, the transformant according to the present invention may produce an interleukin-2 analog when appropriate culture conditions are created depending on the host cell, and the interleukin-2 analog produced according to the constitution of the vector and characteristics of the host cell may be secreted in the cytoplasm or periplasmic space of the host cell or extracellularly.

A protein expressed inside or outside the host cell may be purified by way of a conventional method. Examples of the purification method may include salting-out (e.g., ammonium sulfate precipitation, sodium phosphate precipitation, *etc.),* solvent precipitation *(e.g.,* protein fraction precipitation using acetone or ethanol, *etc.),* dialysis, gel filtration, ion exchange, or chromatography such as reversed column chromatography, ultrafiltration, and the like, and these methods may be applied alone or in combination.

In a specific embodiment of the present invention, a method for preparing an interleukin-2 analog may include:
(a) expressing the interleukin-2 analog; and
(b) isolating the expressed interleukin-2 analog.

In a specific embodiment of the present invention, the method may further include the following steps in order to isolate and purify, from the transformant, an interleukin-2 analog expressed in the form of an inclusion body:
b-1) harvesting the transformant from the culture in step a) and lysing the transformant;
b-2) recovering the expressed interleukin-2 analog from the lysed cell lysate and refolding the interleukin-2 analog; and
b-3) purifying the refolded interleukin-2 analog by way of size-exclusion chromatography.

Another aspect for implementing the present invention provides a method for preparing the interleukin-2 analog by peptide synthesis. As for such peptide synthesis, the interleukin-2 analog can be prepared without limitation by using a known peptide synthesis method since the sequence of the interleukin-2 analog of the present invention is provided.

The alteration of the interleukin-2 analog may be an alteration of at least one amino acid selected from the group consisting of amino acids at positions 61, 62, 68, and 125 in the natural interleukin-2. Such an interleukin-2 analog is as described above.

Another aspect for implementing the present invention provides a method for changing binding affinity of interleukin-2 to interleukin-2 alpha receptor and/or interleukin-2 beta receptor compared with natural interleukin-2, the method including altering at least one amino acid in the natural interleukin-2. The changing of the binding affinity may be increasing the binding affinity to interleukin-2 alpha receptor and decreasing or increasing the binding affinity to interleukin-2 beta receptor.

The interleukin-2 analog, binding affinity, and alteration are as described above.

Hereinafter, the present invention will be described in more detail with reference to exemplary embodiments. These exemplary embodiments are given for specifically illustrating the present invention, and the scope of the present invention is not limited to these exemplary embodiments.

### Example 1: Construction of natural interleukin-2 and interleukin-2 analog expression vectors

In order to construct a natural interleukin-2 expression vector encoding 133 amino acids, the interleukin-2 synthesized on the basis of the reported interleukin-2 sequence (NM_000586.3) was cloned into pET-22b vector (Novagen). In addition, novel interleukin-2 analogs were prepared by altering amino acids of the interleukin-2 while the interleukin-2 was used as a template.

The changed amino acid sequence and analog name of each of the analogs are shown in Table 1 below. In order to prepare these interleukin-2 analogs, forward (F) and reverse (R) primers were synthesized (Table 2), and then each analog gene was amplified by performing PCR.

The primer sequences for each analog in Table 2 represent forward and reverse primers, respectively. The bold text in Table 2 below indicates alteration positions.

**TABLE 1**

| Types, alteration positions, and changed sequences of interleukin-2 analogs | |
|---|---|
| Analog | Alteration position and changed sequence |
| Analog #1 | delA1, C125S |
| Analog #2 | delA1, C125S, E61D |
| Analog #3 | delA1, C125S, E61Q |
| Analog #4 | delA1, C125S, E62Q |
| Analog #5 | delA1, C125S, E68D |

| | |
|---|---|
| delA1 indicates a deletion of alanine as the amino acid at position 1 in interleukin-2. | |

**TABLE 2**

| Primers for interleukin-2 analog amplification | | |
|---|---|---|
| Analog | Sequence | Sequence number |
| Analog #1 | F: CGCCATATGCCTACTTCAAGTTCTACAAAGAA AA | 1 |
| | R: CGGGATCCTCAAGTCAGTGTTGAGATGATGC TTT | 2 |
| | F: AGATGGATTACCTTTAGTCAAAGCATCATCTC A | 3 |
| | R: TGAGATGATGCTTTGACTAAAGGTAATCCATC T | 4 |
| Analog #2 | F: CTTCAGTGTCTAGAAGACGAACTCAAACCTC TG | 5 |
| | R: CAGAGGTTTGAGTTCGTCTTCTAGACACTGA | 6 |
| | AG | |
| Analog #3 | F: CTTCAGTGTCTAGAACAGGAACTCAAACCTC TG | 7 |
| | R: CAGAGGTTTGAGTTCCTGTTCTAGACACTGA AG | 8 |
| Analog #4 | F: CTTCAGTGTCTAGAAGAACAGCTCAAACCTC TG | 9 |
| | R: CAGAGGTTTGAGCTGTTCTTCTAGACACTGA AG | 10 |
| Analog #5 | F: CTCAAACCTCTGGAGGACGTGCTAAATTTAG CT | 11 |
| | R: AGCTAAATTTAGCACGTCCTCCAGAGGTTTGA G | 12 |

PCR conditions for the interleukin-2 analog amplification were 16 cycles of 95°C for 30 seconds, 55°C for 60 seconds, and 65°C for 6.5 minutes. In order to investigate whether amino acids at desired positions were normally altered, the mutagenesis products obtained in the above conditions were sequenced, and it was verified that the interleukin-2 analogs had substitutions with changed sequences at desired alteration positions. The expression vectors thus obtained were named pET22b-interleukin-2 analog 1 to 5.

The DNA sequence and protein sequence of each of the interleukin-2 analog 1 to 5 are shown in Table 3 below. The bold text in Table 3 below indicates alteration positions.

**TABLE 3**

| DNA sequences and protein sequences of interleukin-2 analogs | | | |
|---|---|---|---|
| Analog | Sequence | | SEQ ID NO |
| Analog #1 | DNA | CC TACTTCAAGT TCTACAAAGA | 13 |
| | | AAACACAGCT ACAACTGGAG CATTTACTGC TGGATTTACA GATGATTTTG AATGGAATTA ATAATTACAA GAATCCCAAA CTCACCAGGA TGCTCACATT TAAGTTTTAC ATGCCCAAGA AGGCCACAGA ACTGAAACAT CTTCAGTGTC TAGAAGAAGA ACTCAAACCT CTGGAGGAAG TGCTAAATTT AGCTCAAAGC AAAAACTTTC ACTTAAGACC CAGGGACTTA ATCAGCAATA TCAACGTAAT AGTTCTGGAA CTAAAGGGAT CTGAAACAAC ATTCATGTGT GAATATGCTG ATGAGACAGC AACCATTGTA GAATTTCTGA ACAGATGGAT TACCTTTAGT CAAAGCATCA TCTCAACACT GACTTGA | |
| | Protein | PTSSSTKKTQ LQLEHLLLDL QMILNGINNY KNPKLTRMLT FKFYMPKKAT ELKHLQCLEE ELKPLEEVLN LAQSKNFHLR PRDLISNINV IVLELKGSET TFMCEYADET ATIVEFLNRW ITFSQSIIST LT | 14 |
| Analog #2 | DNA | CC TACTTCAAGT TCTACAAAGA AAACACAGCT ACAACTGGAG CATTTACTGC TGGATTTACA GATGATTTTG AATGGAATTA ATAATTACAA GAATCCCAAA CTCACCAGGA TGCTCACATT TAAGTTTTAC ATGCCCAAGA AGGCCACAGA ACTGAAACAT CTTCAGTGTC TAGAAGACGA ACTCAAACCT CTGGAGGAAG TGCTAAATTT AGCTCAAAGC AAAAACTTTC ACTTAAGACC CAGGGACTTA ATCAGCAATA TCAACGTAAT AGTTCTGGAA CTAAAGGGAT CTGAAACAAC ATTCATGTGT GAATATGCTG ATGAGACAGC AACCATTGTA GAATTTCTGA ACAGATGGAT TACCTTTAGT CAAAGCATCA TCTCAACACT GACTTGA | 15 |
| | Protein | PTSSSTKKTQ LQLEHLLLDL QMILNGINNY KNPKLTRMLT FKFYMPKKAT ELKHLQCLED ELKPLEEVLN LAQSKNFHLR PRDLISNINV IVLELKGSET TFMCEYADET ATIVEFLNRW ITFSQSIIST LT | 16 |
| Analog #3 | DNA | CC TACTTCAAGT TCTACAAAGA AAACACAGCT ACAACTGGAG CATTTACTGC TGGATTTACA GATGATTTTG AATGGAATTA ATAATTACAA GAATCCCAAA CTCACCAGGA TGCTCACATT TAAGTTTTAC ATGCCCAAGA AGGCCACAGA ACTGAAACAT CTTCAGTGTC | 17 |
| | | TAGAACAGGA ACTCAAACCT CTGGAGGAAG TGCTAAATTT AGCTCAAAGC AAAAACTTTC ACTTAAGACC CAGGGACTTA ATCAGCAATA TCAACGTAAT AGTTCTGGAA CTAAAGGGAT CTGAAACAAC ATTCATGTGT GAATATGCTG ATGAGACAGC AACCATTGTA GAATTTCTGA ACAGATGGAT TACCTTTAGT CAAAGCATCA TCTCAACACT GACTTGA | |
| | Protein | PTSSSTKKTQ LQLEHLLLDL QMILNGINNY KNPKLTRMLT FKFYMPKKAT ELKHLQCLEQ ELKPLEEVLN LAQSKNFHLR PRDLISNINV IVLELKGSET TFMCEYADET ATIVEFLNRW ITFSQSIIST LT | 18 |
| Analog #4 | DNA | CC TACTTCAAGT TCTACAAAGA AAACACAGCT ACAACTGGAG CATTTACTGC TGGATTTACA GATGATTTTG AATGGAATTA ATAATTACAA GAATCCCAAA CTCACCAGGA TGCTCACATT TAAGTTTTAC ATGCCCAAGA AGGCCACAGA ACTGAAACAT CTTCAGTGTC TAGAAGAACA GCTCAAACCT CTGGAGGAAG TGCTAAATTT AGCTCAAAGC AAAAACTTTC ACTTAAGACC CAGGGACTTA ATCAGCAATA TCAACGTAAT AGTTCTGGAA CTAAAGGGAT CTGAAACAAC ATTCATGTGT GAATATGCTG ATGAGACAGC AACCATTGTA GAATTTCTGA ACAGATGGAT TACCTTTAGT CAAAGCATCA TCTCAACACT GACTTGA | 19 |
| | Protein | PTSSSTKKTQ LQLEHLLLDL QMILNGINNY KNPKLTRMLT FKFYMPKKAT ELKHLQCLEE QLKPLEEVLN LAQSKNFHLR PRDLISNINV IVLELKGSET TFMCEYADET ATIVEFLNRW ITFSQSIIST LT | 20 |
| Analog #5 | DNA | CC TACTTCAAGT TCTACAAAGA AAACACAGCT ACAACTGGAG CATTTACTGC TGGATTTACA GATGATTTTG AATGGAATTA ATAATTACAA GAATCCCAAA CTCACCAGGA TGCTCACATT TAAGTTTTAC ATGCCCAAGA AGGCCACAGA ACTGAAACAT CTTCAGTGTC TAGAAGAAGA CCTCAAACCT CTGGAGGAAG TGCTAAATTT AGCTCAAAGC AAAAACTTTC ACTTAAGACC CAGGGACTTA ATCAGCAATA TCAACGTAAT AGTTCTGGAA CTAAAGGGAT CTGAAACAAC ATTCATGTGT GAATATGCTG | 21 |
| | | ATGAGACAGC AACCATTGTA GAATTTCTGA ACAGATGGAT TACCTTTAGT CAAAGCATCA TCTCAACACT GACTTGA | |
| | Protein | PTSSSTKKTQ LQLEHLLLDL QMILNGINNY KNPKLTRMLT FKFYMPKKAT ELKHLQCLEE ELKPLEDVLN LAQSKNFHLR PRDLISNINV IVLELKGSET TFMCEYADET ATIVEFLNRW ITFSQSIIST LT | 22 |

### Example 2: Expression of interleukin-2 analogs

By using the expression vectors prepared in Example 1, recombinant interleukin-2 analogs were expressed under the control of the T7 promoter. *E. coli* BL21DE3 (E. *coli* B F-dcm ompT hsdS(rB-mB-) gal λ(DE3): Novagen) as an *E. coli* strain for expression was transformed with each of the recombinant interleukin-2 analog expression vectors. A transformation method recommended by Novagen was employed. Each single colony transformed with each recombinant expression vector was taken, inoculated into 2X Luria broth medium containing ampicillin (50 µg/mL), and cultured at 37°C for 15 hours. The recombinant stain culture and 2X LB medium containing 30% glycerol were mixed at a ratio of 1:1 (v/v), and 1 mL of the mixture was dispensed into each cryo-tube and stored at -150°C. This was used as a cell stock for producing each recombinant protein.

For the expression of recombinant interleukin-2 analogs, one vial of each cell stock was dissolved, inoculated in 500 mL of 2X LB, and incubated with shaking at 37°C for 14-16 hours. The incubation was terminated when the OD value at 600 nm reached 4.0 or higher, and the culture was used as a seed culture. The seed culture was inoculated into 1.6 L of a fermentation medium by using a 5 L fermenter (Bioflo-320, NBS, USA), and initial fermentation was started. The culture conditions were a temperature of 37°C, an air volume of 2.0 L/min (1 vvm), and a stirring rate of 650 rpm, and 30% ammonia water was used to maintain pH 6.70. As for the progression of the fermentation, when the nutrients in the culture medium were limited, an additional medium (feeding solution) was added, followed by fed-batch culture. The growth of the strain was monitored through OD values, and when the OD value reached 70 or higher, IPTG at a final concentration of 50 0 µM was introduced. The culture was further continued for about 23-25 hours after IPTG introduction, and after the termination of the culture, the recombinant strain was harvested by using a centrifuge and stored at -80°C until use.

### Example 3: Extraction and refolding of interleukin-2 analogs

In order that each of the interleukin-2 analogs from the interleukin-2 analog-expressed *E. coli* obtained in Example 2 above was converted into a soluble form, the cells were lysed and refolded. Cell pellets corresponding to a 100 mL aliquot of the culture were suspended in 1-200 mL of a lysis buffer (20 mM Tris-HCI pH 9.0, 1 mM EDTA pH 9.0, 0.2 M NaCl, 0.5% Triton X-100), and then recombinant *E. coli was* lysed at 15,000 psi by using a microfluidizer. After centrifugation at 13,900 *g* for 30 minutes, the supernatant was discarded, and the pellets were washed with 400 mL of a first wash buffer (50 mM Tris-HCI pH 8.0, 5 mM EDTA pH 9.0). After centrifugation under the same conditions as above, the supernatant was discarded, and the pellets were washed with 400 mL of a second wash buffer (50 mM Tris-HCI pH 8.0, 5 mM EDTA pH 9.0, 2% Triton X-100). After centrifugation under the same conditions as above, the supernatant was discarded, and the pellets were washed with 400 mL of a third wash buffer (50 mM Tris-HCI pH 8.0, 5 mM EDTA pH 9.0, 1% sodium deoxycholorate). After centrifugation under the same conditions as above, the supernatant was discarded, and the pellets were washed with 400 mL of a fourth wash buffer (50 mM Tris-HCI pH 8.0, 5 mM EDTA pH 9.0, 1 M NaCl). After centrifugation under the same conditions and washing, *E. coli* inclusion body pellets were obtained. The washed inclusion body pellets were re-suspended in 400 mL of a solubilizing/reducing buffer (6 M guanidine, 100 mM Tris pH 8.0, 2 mM EDTA pH 9.0, 50 mM DTT), and stirred at 50°C for 30 minutes. After 100 mL of distilled water was added to the solubilized/reduced interleukin-2 analog to dilute 6 M guanidine to 4.8 M guanidine, centrifugation at 13,900 g was conducted for 30 minutes, and then the pellets were discarded, and only the solution was obtained. After 185.7 mL of distilled water was further added to the diluted solution to dilute 4.8 M guanidine to 3.5 M guanidine, pH was adjusted to 5.0 by using 100% acetic acid. The pH-adjusted solution was stirred at room temperature for 1 hour. The solution with precipitated impurities was centrifuged at 13,900 g for 30 minutes, and the supernatant was discarded, and then the pellets were washed with the last wash buffer (3.5 M guanidine, 20 mM sodium acetate pH 5.0, 5 mM DTT). Through centrifugation under the same conditions as above, pellets were obtained. The washed interleukin-2 analog was dissolved in 400 mL of a refolding buffer (6 mM guanidine, 100 mM Tris pH 8.0, 0.1 mM CuCl₂). The mixture solution was subjected to refolding by stirring at 4°C for 15-24 hours.

### Example 4: Size-exclusion column chromatography

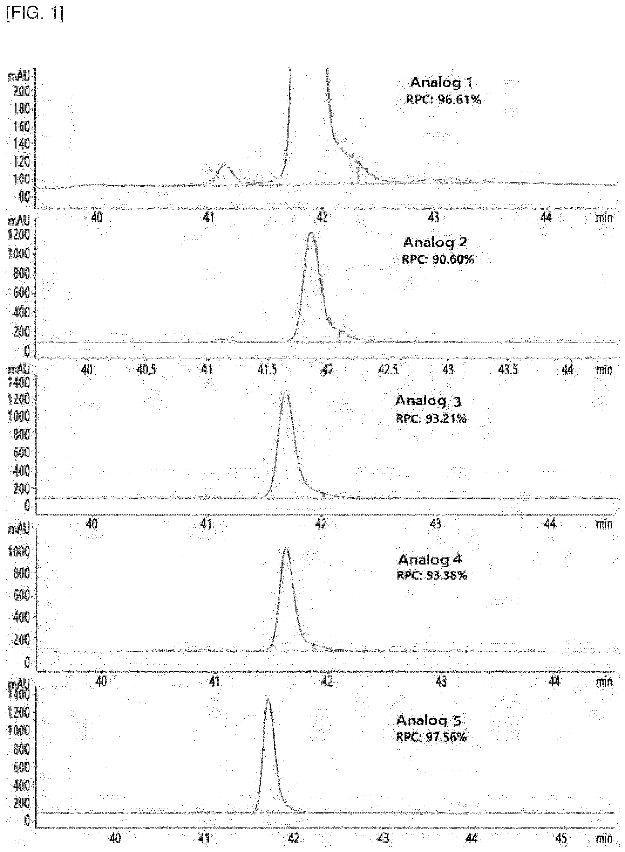

The interleukin-2 analog refolded solution obtained in Example 3 was concentrated to 1 mL or less in order to be purified through the application to a size-exclusion column. The column was equilibrated with a buffer (2 M guanidine, 100 mM Tris pH 8.0) before the introduction of the refolded solution, and after the introduction of the refolded solution, elution was conducted by flowing a buffer through the column. The eluted sample contained guanidine, and thus exchanged with a stabilization solution (10 mM sodium acetate pH 4.5, 5% trehalose), and then purity was determined through RP-HPLC and peptide mapping analysis (FIG. 1).

### Example 5: Evaluation of receptor binding affinity of interleukin-2 analogs

In order to determine binding affinity of the interleukin-2 analogs obtained in Example 4 to each of interleukin-2 alpha receptor and interleukin-2 beta receptor, surface plasmon resonance (SPR, BIACORE T200, GE Healthcare) was used.

First, an anti-human immunoglobulin antibody (Abcam, #ab97221) was immobilized to a CM5 chip (GE Healthcare) by about 5,000 resonance units (RU) through amine coupling, and then the interleukin-2 alpha receptor (SYMANSIS, #4102H) or interleukin-2 beta receptor (SYMANSIS, #4122H) linked to the human immunoglobulin Fc region was finally immobilized to the immunoglobulin antibody by binding using an antigen-antibody binding response. Then, each of the recombinant interleukin-2 analogs prepared above were diluted to have different concentrations, which were allowed to flow through the CM5 chips on which the interleukin-2 receptor was finally immobilized, to thereby determine the binding affinity to each of the interleukin-2 receptors. The binding affinity was determined using the association rate (Kₐ) and the dissociation rate (K_{d}), wherein the association rate was measured by flowing each of the interleukin-2 analogs at a flow rate of 10 µL/min for 3 minutes, and the dissociation rate from each of the interleukin-2 receptors was measured by flowing only an experimental buffer at the same flow rate for the same time. Upon completion of the measurement, the binding affinity to the receptors was evaluated according to 1:1 binding fitting model in Biaevaluation program.

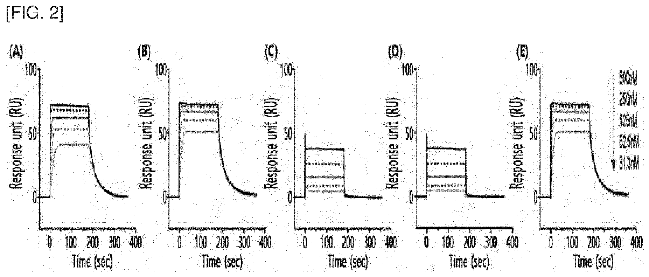

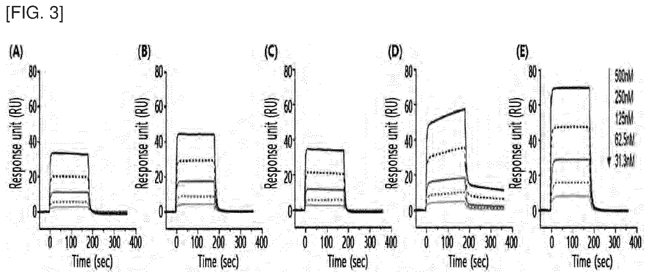

As shown in the test results (FIG. 2, FIG. 3, and Table 4), the binding affinity of interleukin-2 analogs 2, 3, 4, and 5 to interleukin-2 alpha receptor was measured to be higher compared with the natural interleukin-2 (analog 1) used as a control, while the above analogs 2, 3, 4, and 5 had an increase or decrease in binding affinity to interleukin-2 beta receptor compared with the natural interleukin-2. These results confirmed that the amino acids at positions 61, and/or 62, and/or 68 of the interleukin-2 had an influence on the binding affinity to interleukin-2 alpha receptor or beta receptor. These results suggest that the substitutions of corresponding amino acids can change the binding affinity to interleukin-2 receptor.

**TABLE 4**

| Binding affinity of interleukin-2 analogs to interleukin-2 alpha receptor and beta receptor | | | | |
|---|---|---|---|---|
| Interleukin-2 receptor | Test substance | Kₐ (1/M/s, ×10⁵) | K_{d} (1/s, × 10⁻³) | K_{D} (nM) |
| Alpha receptor | Natural IL-2 | 41.2 ± 5.2 | 70.2 ± 6.2 | 17.1 ± 0.7 |
| | Analog 02 | 85.3 ± 22.8 | 60.0 ± 14.1 | 7.1 ± 0.2 |
| | Analog 03 | 94.2 ± 109.9 | 206.5 ± 128.0 | 54.3 ± 43.6 |
| | Analog 04 | 0.4 ± 0.0 | 21.4 ± 3.0 | 478.7 ± 57.5 |
| | Analog 05 | 101.5 ± 15.0 | 26.3 ± 3.4 | 2.6 ± 0.0 |
| Beta receptor | Natural IL-2 | N/A | N/A | 242.3 ± 27.6 |
| | Analog 02 | N/A | N/A | 124.9 ± 5.1 |
| | Analog 03 | N/A | N/A | 108.1 ± 94.0 |
| | Analog 04 | N/A | N/A | 533.4 ± 166.9 |
| | Analog 05 | N/A | N/A | 1124.2 ± 2.6 |

While the present invention has been described with reference to the particular illustrative embodiments, a person skilled in the art to which the present invention pertains can understand that the present invention may be embodied in other specific forms without departing from the technical spirit or essential characteristics thereof. Therefore, the exemplary embodiments described above should be construed as being exemplified and not limiting the present disclosure. The scope of the present invention should be construed such that all changes or modifications derived from the definitions and scope of the claims and their equivalents fall within the scope of the invention.

## Claims

1. An interleukin-2 analog having changes in binding affinity to interleukin-2 alpha receptor and binding affinity to interleukin-2 beta receptor compared with natural interleukin-2, wherein the interleukin-2 analog has an alteration of at least one amino acid in the natural interleukin-2.

2. The interleukin-2 analog of claim 1, wherein the changes in binding affinity are an increase in binding affinity to interleukin-2 alpha receptor and a decrease or increase in binding affinity to interleukin-2 beta receptor.

3. The interleukin-2 analog of claim 2, wherein the decrease or increase in binding affinity to interleukin-2 beta receptor shows 10% to 200% compared to the binding affinity of the natural interleukin-2.

4. The interleukin-2 analog of claim 1, wherein the alteration is selected from the group consisting of substitution, addition, deletion, modification, and a combination thereof of at least one amino acid in the natural interleukin-2.

5. The interleukin-2 analog of claim 1, wherein the interleukin-2 analog has an alteration of at least one amino acid to another amino acid, the at least one amino acid being selected from the group consisting of the amino acids at positions 1, 61, 62, 68, and 125 in the natural interleukin-2.

6. The interleukin-2 analog of claim 1, wherein the interleukin-2 analog is any one of the following analogs:
(a) an interleukin-2 analog having an alteration of the amino acid at position 1 in the natural interleukin-2;
(b) an interleukin-2 analog having an alteration of the amino acid at position 61 to another amino acid in the natural interleukin-2;
(c) an interleukin-2 analog having an alteration of the amino acid at position 62 to another amino acid in the natural interleukin-2;
(d) an interleukin-2 analog having an alteration of the amino acid at position 68 to another amino acid in the natural interleukin-2; and
(e) an interleukin-2 analog having an alteration of the amino acid at position 125 to another amino acid in the natural interleukin-2.

7. The interleukin-2 analog of claim 1, wherein the interleukin-2 analog is any one of the following analogs:
(a) an interleukin-2 analog having a deletion of the amino acid at position 1 and a substitution of the amino acid at position 125 with another amino acid in the natural interleukin-2;
(b) an interleukin-2 analog having a deletion of the amino acid at position 1 and substitutions of the amino acids at positions 125 and 61 with other amino acids in the natural interleukin-2;
(c) an interleukin-2 analog having a deletion of the amino acid at position 1 and substitutions of the amino acids at positions 125 and 62 with other amino acids in the natural interleukin-2; and
(d) an interleukin-2 analog having a deletion of the amino acid at position 1 and substitutions of the amino acids at positions 125 and 68 with other amino acids in the natural interleukin-2.

8. The interleukin-2 analog of claim 1, wherein the interleukin-2 analog is any one of the following analogs:
(a) an interleukin-2 analog having a deletion of the amino acid at position 1, a substitution of the amino acid at position 125 with serine, and a substitution of the amino acid at position 61 with aspartic acid in the natural interleukin-2;
(b) an interleukin-2 analog having a deletion of the amino acid at position 1, a substitution of the amino acid at position 125 with serine, and a substitution of the amino acid at position 61 with glutamine in the natural interleukin-2;
(c) an interleukin-2 analog having a deletion of the amino acid at position 1, a substitution of the amino acid at position 125 with serine, and a substitution of the amino acid at position 62 with glutamine in the natural interleukin-2; and
(d) an interleukin-2 analog having a deletion of the amino acid at position 1, a substitution of the amino acid at position 125 with serine, and a substitution of the amino acid at position 68 with aspartic acid in the natural interleukin-2.

9. The interleukin-2 analog of claim 1, wherein the interleukin-2 analog is selected from the group consisting of SEQ ID NOS: 16, 18, 20, and 22.

10. A nucleic acid encoding the interleukin-2 analog of any one of claims 1 to 9.

11. A recombinant expression vector comprising the nucleic acid of claim 10.

12. A non-human transformant comprising the recombinant expression vector of claim 11.

13. A method for changing binding affinity to interleukin-2 alpha receptor and interleukin-2 beta receptor compared with natural interleukin-2, the method comprising altering at least one amino acid in the natural interleukin-2.

14. The method of claim 13, wherein the changing of the binding affinity is increasing the binding affinity to interleukin-2 alpha receptor and decreasing or increasing the binding affinity to interleukin-2 beta receptor.

15. The method of claim 13, wherein the altering is altering at least one amino acid to another amino acid, the at least one amino acid being selected from the group consisting of amino acids at positions 1, 61, 62, 68, and 125 in the natural interleukin-2.

16. The method of claim 13, wherein the altering is:
(a) deleting the amino acid at position 1 and substituting the amino acids at positions 125 and 61 with other amino acids in the natural interleukin-2;
(c) deleting the amino acid at position 1 and substituting the amino acids at positions 125 and 62 with other amino acids in the natural interleukin-2; or
(d) deleting the amino acid at position 1 and substituting the amino acids at positions 125 and 68 with other amino acids in the natural interleukin-2.


<110>    HANMI PHARM. CO., LTD.

<120>    Novel immune suppressing IL-2 analogs

<130>    OPA19228-PCT

<150>    KR 10-2018-0167797
<151>    2018-12-21

<160>    23

<170>    KoPatentIn 3.0

<210>    1
<211>    34
<212>    DNA
<213>    Artificial Sequence

<220>
<223>    F primer of analog 1


<400>    1
cgccatatgc ctacttcaag ttctacaaag aaaa                                    34


<210>    2
<211>    34
<212>    DNA
<213>    Artificial Sequence

<220>
<223>    R primer of analog 1


<400>    2
cgggatcctc aagtcagtgt tgagatgatg cttt                                    34


<210>    3
<211>    33
<212>    DNA
<213>    Artificial Sequence

<220>
<223>    F primer of analog 1


<400>    3
agatggatta cctttagtca aagcatcatc tca                                     33


<210>    4
<211>    33
<212>    DNA
<213>    Artificial Sequence

<220>
<223>    R primer of analog 1


<400>    4
tgagatgatg ctttgactaa aggtaatcca tct                                     33

<210>	5
<211>	33
<212>	DNA
<213>	Artificial Sequence

<220>
<223>	F primer of analog 2


<400>	5
cttcagtgtc tagaagacga actcaaacct ctg	33


<210>	6
<211>	33
<212>	DNA
<213>	Artificial Sequence

<220>
<223>	R primer of analog 2


<400>	6
cagaggtttg agttcgtctt ctagacactg aag	33


<210>	7
<211>	33
<212>	DNA
<213>	Artificial Sequence

<220>
<223>	F primer of analog 3


<400>	7
cttcagtgtc tagaacagga actcaaacct ctg	33


<210>	8
<211>	33
<212>	DNA
<213>	Artificial Sequence

<220>
<223>	R primer of analog 3


<400>	8
cagaggtttg agttcctgtt ctagacactg aag	33


<210>	9
<211>	33
<212>	DNA
<213>	Artificial Sequence

<220>
<223>	F primer of analog 4

<400>    9
cttcagtgtc tagaagaaca gctcaaacct ctg                                    33


<210>    10
<211>    33
<212>    DNA
<213>    Artificial Sequence

<220>
<223>    R primer of analog 4


<400>    10
cagaggtttg agctgttctt ctagacactg aag                                    33


<210>    11
<211>    33
<212>    DNA
<213>    Artificial Sequence

<220>
<223>    F primer of analog 5


<400>    11
ctcaaacctc tggaggacgt gctaaattta gct                                    33


<210>    12
<211>    33
<212>    DNA
<213>    Artificial Sequence

<220>
<223>    R primer of analog 5


<400>    12
agctaaattt agcacgtcct ccagaggttt gag                                    33


<210>    13
<211>    399
<212>    DNA
<213>    Artificial Sequence

<220>
<223>    Analog 1


<400>    13
cctacttcaa gttctacaaa gaaaacacag ctacaactgg agcatttact gctggattta      60

cagatgattt tgaatggaat taataattac aagaatccca aactcaccag gatgctcaca     120

tttaagtttt acatgcccaa gaaggccaca gaactgaaac atcttcagtg tctagaagaa     180

gaactcaaac ctctggagga agtgctaaat ttagctcaaa gcaaaaactt tcacttaaga     240

cccagggact taatcagcaa tatcaacgta atagttctgg aactaaaggg atctgaaaca     300

acattcatgt gtgaatatgc tgatgagaca gcaaccattg tagaatttct gaacagatgg 360

attaccttta gtcaaagcat catctcaaca ctgacttga 399

<210> 14
<211> 132
<212> PRT
<213> Artificial Sequence

<220>
<223> Analog 1

<400> 14
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Glu Glu Leu Lys Pro
    50                  55                  60

Leu Glu Glu Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

<210> 15
<211> 399
<212> DNA
<213> Artificial Sequence

<220>
<223> Analog 2

<400> 15
cctacttcaa gttctacaaa gaaaacacag ctacaactgg agcatttact gctggattta 60

cagatgattt tgaatggaat taataattac aagaatccca aactcaccag gatgctcaca 120

tttaagtttt acatgcccaa gaaggccaca gaactgaaac atcttcagtg tctagaagac 180

gaactcaaac ctctggagga agtgctaaat ttagctcaaa gcaaaaactt tcacttaaga 240

cccagggact taatcagcaa tatcaacgta atagttctgg aactaaaggg atctgaaaca 300

acattcatgt gtgaatatgc tgatgagaca gcaaccattg tagaatttct gaacagatgg     360

attaccttta gtcaaagcat catctcaaca ctgacttga                           399

<210>    16
<211>    132
<212>    PRT
<213>    Artificial Sequence

<220>
<223>    Analog 2

<400>    16
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Asp Glu Leu Lys Pro
    50                  55                  60

Leu Glu Glu Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

<210>    17
<211>    399
<212>    DNA
<213>    Artificial Sequence

<220>
<223>    Analog 3

<400>    17
cctacttcaa gttctacaaa gaaaacacag ctacaactgg agcatttact gctggattta      60

cagatgattt tgaatggaat taataattac aagaatccca aactcaccag gatgctcaca     120

tttaagtttt acatgcccaa gaaggccaca gaactgaaac atcttcagtg tctagaacag     180

gaactcaaac ctctggagga agtgctaaat ttagctcaaa gcaaaaactt tcacttaaga     240

cccagggact taatcagcaa tatcaacgta atagttctgg aactaaaggg atctgaaaca     300

acattcatgt gtgaatatgc tgatgagaca gcaaccattg tagaatttct gaacagatgg 360

attaccttta gtcaaagcat catctcaaca ctgacttga 399

<210> 18
<211> 132
<212> PRT
<213> Artificial Sequence

<220>
<223> Analog 3

<400> 18
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Gln Glu Leu Lys Pro
    50                  55                  60

Leu Glu Glu Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

<210> 19
<211> 399
<212> DNA
<213> Artificial Sequence

<220>
<223> Analog 4

<400> 19
cctacttcaa gttctacaaa gaaaacacag ctacaactgg agcatttact gctggattta 60

cagatgattt tgaatggaat taataattac aagaatccca aactcaccag gatgctcaca 120

tttaagtttt acatgcccaa gaaggccaca gaactgaaac atcttcagtg tctagaagaa 180

cagctcaaac ctctggagga agtgctaaat ttagctcaaa gcaaaaactt tcacttaaga 240

cccagggact taatcagcaa tatcaacgta atagttctgg aactaaaggg atctgaaaca 300

acattcatgt gtgaatatgc tgatgagaca gcaaccattg tagaatttct gaacagatgg 360

attaccttta gtcaaagcat catctcaaca ctgacttga 399

<210> 20
<211> 132
<212> PRT
<213> Artificial Sequence

<220>
<223> Analog 4

<400> 20
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Glu Gln Leu Lys Pro
    50                  55                  60

Leu Glu Glu Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

<210> 21
<211> 399
<212> DNA
<213> Artificial Sequence

<220>
<223> Analog 5

<400> 21
cctacttcaa gttctacaaa gaaaacacag ctacaactgg agcatttact gctggattta 60

cagatgattt tgaatggaat taataattac aagaatccca aactcaccag gatgctcaca 120

tttaagtttt acatgcccaa gaaggccaca gaactgaaac atcttcagtg tctagaagaa 180

gacctcaaac ctctggagga agtgctaaat ttagctcaaa gcaaaaactt tcacttaaga 240

cccagggact taatcagcaa tatcaacgta atagttctgg aactaaaggg atctgaaaca 300

acattcatgt gtgaatatgc tgatgagaca gcaaccattg tagaatttct gaacagatgg     360

attaccttta gtcaaagcat catctcaaca ctgacttga                           399

<210>    22
<211>    132
<212>    PRT
<213>    Artificial Sequence

<220>
<223>    Analog 5

<400>    22
Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Glu Glu Leu Lys Pro
    50                  55                  60

Leu Glu Asp Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

<210>    23
<211>    133
<212>    PRT
<213>    Homo sapiens

<400>    23